# EUROPEAN PATENT APPLICATION

(11) **EP 4 678 120 A1**
(43) Date of publication of application: **14.01.2026**
(21) Application number: 24767239.7
(22) Date of filing: 08.03.2024
(51) Int. Cl.: A61B 17/00

(54) **ADMINISTERING DEVICE AND MEDICAL IMPLEMENT**

(30) Priority: 09.03.2023 JP 2023036835
(71) Applicant: Seikagaku Corporation, Tokyo 100-0005 (JP)
(72) Inventor: ISHIKAWA, Motoki, Tokyo 100-0005 (JP); HAMADA, Naoki, Tokyo 100-0005 (JP)
(74) Representative: Müller-Boré & Partner Patentanwälte PartG mbB
(86) International application number: PCT/JP2024/008955
(87) International publication number: WO 2024/185874

(57) **Abstract**

An administration device is capable of stably spraying medical powder, which is housed in a chamber, onto a target site even with posture inclination. The administration device includes the chamber having an outlet port through which the medical powder is discharged and having a space where the medical powder is housable, a protrusion protruding into the space from a surface facing the outlet port to the outlet port in the chamber and having an opening in the side surface of the protrusion, and a discharging unit that discharges gas from the opening into the space, through an inlet port of the protrusion communicating with the opening from the side opposite to the tip end of the protrusion.

## Description

### Technical Field

The present invention relates to techniques of an administration device and a medical implement for administering medical powder.

### Background Art

There has been known a device that administers medical powder to an affected area of a patient for the purpose of adhesion prevention, hemostasis, tissue swelling, or the like (Patent Literatures 1 to 4).

For example, an adhesive barrier is used for the purpose of preventing adhesion between a wound site and a surrounding tissue which may adhere to the wound site after a surgery. The adhesion occurs when the tissue surface of the wound site and the surrounding tissue surface are joined or stuck to each other due to leakage/deposition of fibrin-containing exudate on the tissue surface of the wound site, for example. The adhesive barrier is applied or bonded between the wound site and the surrounding tissue which may adhere to the wound site, and functions as a physical partition wall for preventing the joint or sticking between these tissues. Patent Literature 5 discloses an adhesive barrier in the form of powder.

### Citation List

### Patent Literature

Patent Literature 1: WO2003/070110
Patent Literature 2: WO2005/089472
Patent Literature 3: WO2005/072700
Patent Literature 4: WO2010/074949
Patent Literature 5: WO2015/115609

### Summary of Invention

For example, the application of the medical powder onto the affected area by a doctor is performed by operation of an administration device including a chamber housing the medical powder. A surgeon operates the administration device to spray the medical powder, which is housed in the chamber, onto a target site of a patient.

However, depending on an operative procedure or a wound site, it is required that a medical treatment is performed on a patient lying in bed from above in the vertical direction, laterally, or the like. There has been concerns that depending on the relative posture of the administration device operated by the surgeon, a sufficient amount of medical powder for the medical treatment is not sprayed or the medical powder is unevenly sprayed, which may lead to a failure in performing a procedure intended by the surgeon. For the administration device, stable spraying onto the target site has been demanded regardless of the inclination of the chamber housing the medical powder such as the adhesive barrier. An object of the present invention is to provide an administration device capable of stably spraying medical powder, which is housed in a chamber, onto a target site even with posture inclination.

As a result of intensive study conducted by the present inventors, it has been found that the following configuration of the administration device housing the medical powder solves the above-described problems. That is, an administration device according to one embodiment includes a protrusion protruding into a chamber having a space where medical powder such as an adhesive barrier can be housed. In the side surface of the protrusion of the administration device, an opening is formed, which communicates with a discharging unit that discharges gas into the space of the chamber housing the medical powder. With this configuration, the administration device can discharge gas from the discharging unit into the space of the chamber housing the medical powder through the opening provided in the side surface of the protrusion. The gas discharged into the chamber through the opening can form a flow toward an outlet port of the administration device together with the medical powder while swirling the housed medical powder in the air.

A detailed configuration will be described as follows.
[1] An administration device for medical powder, which includes
   a chamber having an outlet port through which the medical powder is discharged and having a space where the medical powder is housable,
   a protrusion protruding into the space from a surface facing the outlet port to the outlet port in the chamber and having an opening in the side surface of the protrusion, and
   a discharging unit that discharges gas from the opening into the space, through an inlet port of the protrusion communicating with the opening from the side opposite to the tip end of the protrusion.
[2] The administration device according to [1], in which
   the protrusion is axisymmetric, and the opening is opened in a circumferential direction about an axial direction in the side surface in the vicinity of the tip end.
[3] The administration device according to [1] or [2], in which
   the shape of the protrusion is a pyramid shape, a conical shape, a rectangular columnar shape, or a circular columnar shape.
[4] The administration device according to any one of [1] to [3], in which
   the protruding end of the protrusion is closed.
[5] The administration device according to any one of [1] to [4], in which
   the opening is opened inside the chamber at a position with a distance of 1/3 or more of a distance between the surface facing the outlet port and an inner surface on the outlet port side in the direction from the surface facing the outlet port toward the outlet port.
[6] The administration device according to any one of [1] to [5], which further includes
   a finger grip on which a finger is hooked when a user grips the administration device.
[7] The administration device according to any one of [1] to [6], in which
   the discharging unit is a pressing pump.
[8] The administration device according to any one of [1] to [7], in which
   the opening includes openings opened at an equal interval at a plurality of locations in the circumferential direction about the axial direction in the side surface in the vicinity of the tip end.
[9] The administration device according to any one of [1] to [8], in which
   the hole area of the opening is 1 mm² or less per hole.
[10] A medical implement including
   the administration device according to any one of [1] to [9], which has the chamber housing the medical powder.
[11] The medical implement according to [10], in which
   the medical powder is an adhesive barrier.
[12] The medical implement according to [10] or [11], which further includes
   a tubular applicator.
[13] A surgical method using the medical implement according to [12], which includes
   inserting at least a tip end portion of the applicator connected to the outlet port into a living body, and
   discharging gas from the tip end portion to spray the medical powder onto a target site of the living body together with the gas.
[14] The surgical method according to [13], in which
   a surgery is a laparotomy or a laparoscopic surgery.
[15] The surgical method according to [13] or [14], in which
   the target site is a peritoneal membrane.

### Brief Description of Drawings

[FIG. 1] FIG. 1 shows views of one example of the configuration of an administration device according to an embodiment.
[FIG. 2] FIG. 2 shows views for describing a state related to use of the administration device according to an embodiment.
[FIG. 3] FIG. 3 shows views for describing the operation posture of the administration device housing medical powder.
[FIG. 4] FIG. 4 shows views for describing the structure of a protrusion of the administration device according to an embodiment.
[FIG. 5] FIG. 5 shows views for describing other structures of the protrusion of the administration device according to an embodiment.
[FIG. 6] FIG. 6 shows views for describing a spraying test when the inclination angle of the administration device according to an embodiment is 0 degrees.
[FIG. 7] FIG. 7 shows views for describing a spraying test when the inclination angle of the administration device according to an embodiment is 25 degrees.
[FIG. 8] FIG. 8 shows views for describing a spraying test when the inclination angle of the administration device according to an embodiment is 40 degrees.

### Description of Embodiments

Hereinafter, an administration device according to an embodiment (hereinafter also referred to as one embodiment or an embodiment) for carrying out the invention will be described with reference to the drawings. The following configuration of the embodiment is an example, and the present administration device is not limited to the configuration of the embodiment. Moreover, the dimensions, materials, shapes, relative arrangements, and the like of components disclosed in the present embodiment are not intended to limit the technical scope of the invention only to these dimensions, materials, shapes, relative arrangements, and the like unless otherwise specified.

According to the present invention, the administration device can be provided, which is capable of stably spraying medical powder, which is housed in a chamber, to a target site even with posture inclination.

In the following description, the medical powder indicates powder to be used for medical purposes. The medical powder includes an adhesive barrier, a hemostatic material, a tissue swelling material, a wound dressing material, and the like used for a surgery and the like. Examples of the adhesive barrier and the tissue swelling material include a crosslinked acidic polysaccharide (e.g., crosslinked chondroitin sulfate) disclosed in Patent Literature 5 and the like. Examples of the hemostatic material include oxidized cellulose, fibrinogen, collagen, gelatin, and the like. Examples of the wound dressing material include alginic acid, carboxymethylcellulose, and the like. Note that the medical powder is not limited to those described above.

In terms of the particle size (diameter) of the medical powder, 95% or more of all particles in mass equivalent preferably have less than 1 mm, and 95% or more of all the particles in mass equivalent more preferably have less than 0.5 mm. Note that the particle size of the medical powder is a value measured according to the Japanese Pharmacopoeia, 18th Edition "3.04 Particle Size Determination Method 2. Analytical Sieving Method".

### (Administration device for Medical Powder)

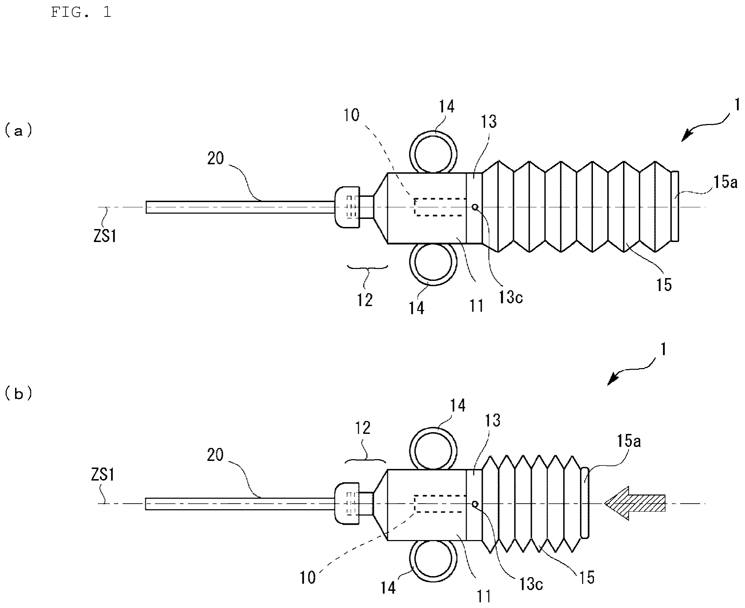

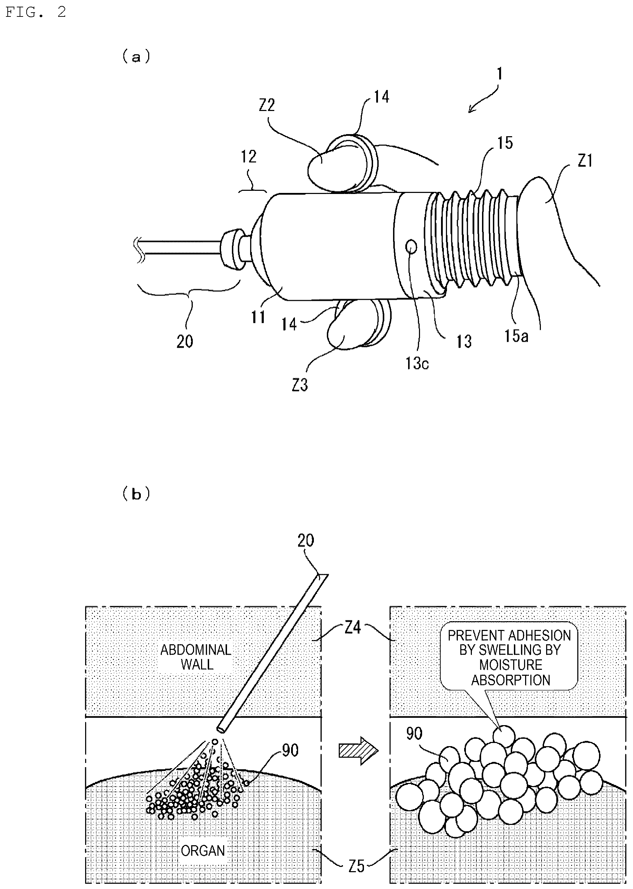

First, the administration device according to the present embodiment will be described with reference to FIGS. 1 and 2. FIG. 1 shows views of one example of the configuration of an administration device 1 according to the present embodiment. As an example, FIG. 1(a) shows a side view of the entire configuration of the administration device 1, and FIG. 1(b) shows a side view of a state of a discharging unit 15 being pressed in an axial direction. In FIGS. 1(a) and 1(b), a dash-dotted line indicates the center axis ZS1 of the administration device 1, and a direction in which the center axis ZS1 extends will be referred to as an axial direction. Note that in FIG. 1, this axial direction is a right-left direction when facing the drawing. FIG. 2 shows views for describing a state related to use of the administration device 1 according to the present embodiment. As an example, FIG. 2(a) shows a perspective view for describing operation of the administration device 1, and FIG. 2(b) shows a view for describing the mechanism of action of an adhesive barrier 90 sprayed as the medical powder.

As shown in FIGS. 1(a) and 1(b), the administration device 1 has a protrusion 10, a chamber 11, an outlet unit 12, a connection unit 13, finger grips 14, and the discharging unit 15, and these components are integrally assembled in the axial direction to form a substantially syringe shape as a whole. In the following description, a direction in which the outlet unit 12 of the administration device 1 is disposed will also be referred to as a tip end direction, and a direction in which the discharging unit 15 is disposed will also be referred to as a base end direction.

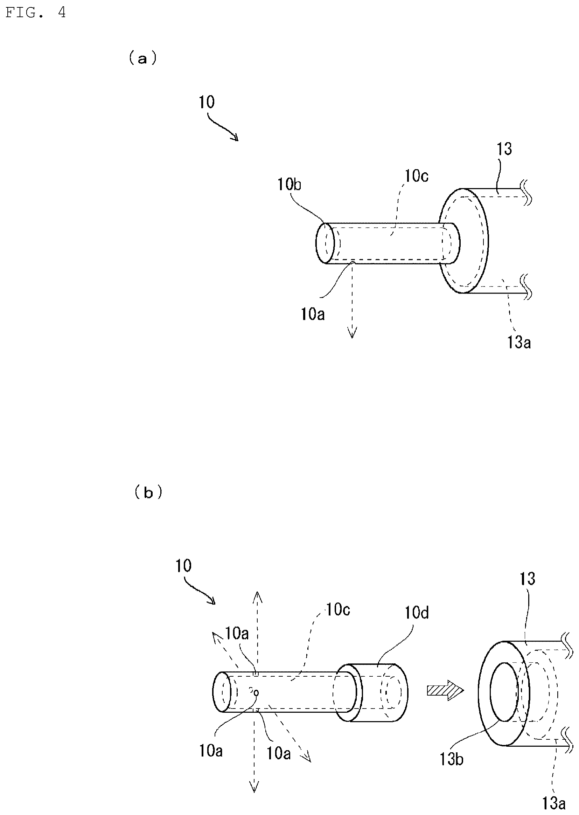

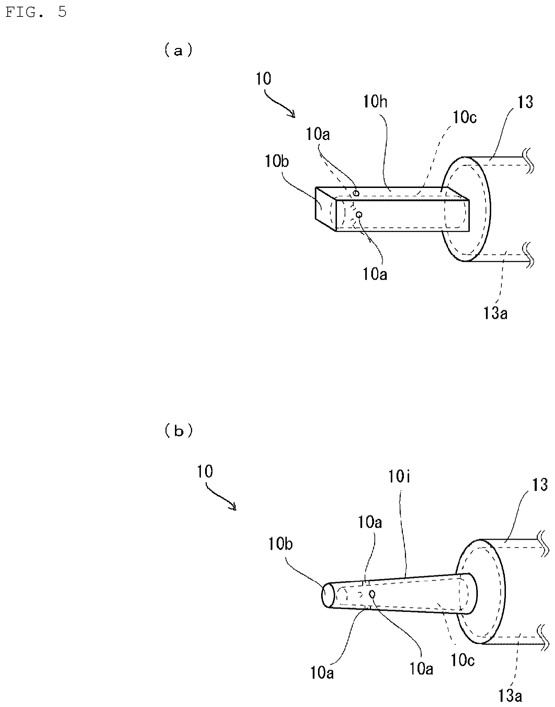

In the administration device 1 according to the present embodiment, the protrusion 10 is formed hollow so as to protrude into the chamber 11 having a space where the medical powder can be housed. The protrusion 10 protrudes from a surface facing an outlet port of the outlet unit 12 to the outlet port in the chamber 11, and in a side surface in the vicinity of the tip end, an opening communicating with the discharging unit 15 is provided in a circumferential direction. The protrusion 10 of the administration device 1 discharges gas, which is discharged through the discharging unit 15 pressed by pressing operation by a user such as a surgeon, into the space of the chamber 11 housing the medical powder through the opening. The gas discharged into the chamber 11 through the opening flows toward the outlet unit 12 together with the medical powder while stirring the housed medical powder, and is discharged onto a target site of a patient through an applicator 20 engaged with the outlet unit. Note that the protrusion 10 will be described in detail with reference to FIGS. 4 and 5.

The chamber 11 is a column-shaped member having a space where the medical powder is housed. The outlet unit 12 is disposed on the tip end side of the chamber 11, and the connection unit 13 is disposed on the base end side of the chamber 11. Chambers 11 with various shapes, sizes, and the like may be employed as necessary according to the type, material, and the like of the medical powder to be housed. The material of the chamber 11 is not limited, and any of materials forming well-known chambers, such as a glass material and a resin material, may be employed. In a case where the material of the chamber 11 is a synthetic resin material, the chamber 11 can be molded in an arbitrary shape by injection molding, extrusion molding, or the like.

The outlet unit 12 is a member communicating with the space of the chamber 11 and having therein a path for discharging the medical powder housed in such a chamber through the outlet port together with the gas. The base end side of the outlet unit 12 is formed in a substantially frustum shape so that the outlet unit 12 can be engaged with the chamber 11, and on the tip end side, is formed in a tubular shape so that the outlet unit 12 can be engaged with the applicator (tubular or tubelike needle) 20 for discharging the medical powder onto the target site. The outlet port of the outlet unit 12 communicates with the inside of the tube of the applicator 20. The base-end-side shape of the outlet unit 12 is only required to be such a shape that the outlet unit 12 can be engaged with the chamber 11. The same also applies to the tip-end-side shape of the outlet unit 12. That is, the tip-end-side shape is only required to be such a shape engageable with the applicator 20. For the engagement between the base end side of the outlet unit 12 and the chamber 11 and the engagement between the tip end side of the outlet unit 12 and the applicator 20, a well-known engagement method such as screwing or interference fit may be employed as necessary according to the materials, shapes, sizes, and the like of these components. For example, the tip-end-side outer peripheral surface of the outlet unit 12 is threaded, and a thread groove to be screwed with the tip-end-side thread of the outlet unit 12 is formed in the base-end-side inner peripheral surface of the applicator 20, and in this manner, these components can be engaged with each other by fitting.

The material of the outlet unit 12 is also not limited, and a well-known material such as a resin material may be employed as necessary. Note that in a case where the material of the outlet unit 12 is the same synthetic resin material as that of the chamber 11, the outlet unit 12 and the chamber 11 may be integrally molded by injection molding, extrusion molding, or the like. The structure of the applicator 20 is not particularly limited as long as the medical powder housed in the chamber 11 can be discharged onto the target site together with the gas, but from the point of view of easy spraying onto the target site and minimal invasiveness, the applicator 20 is preferably a resin member having elasticity.

The tube inner diameter (diameter) of the applicator 20 is not particularly limited as long as the applicator 20 has such a size that the medical powder passes therethrough, but is preferably more than 1 mm and 5 mm or less and more preferably 2 mm or more and 3.5 mm or less.

The connection unit 13 is a member connecting the chamber 11 and the discharging unit 15 to each other. For example, the connection unit 13 is formed, on the tip end side, in such a shape that the connection unit 13 can be engaged with the base end side of the chamber 11, and on the base end side, is formed in such a shape that the connection unit 13 can be engaged with the tip end side of the discharging unit 15. For the connection between the tip end side of the connection unit 13 and the chamber 11 and the connection between the base end side of the connection unit 13 and the discharging unit 15, a well-known engagement method such as screwing or interference fit may be employed as necessary according to the materials, shapes, sizes, and the like of these components. The material of the connection unit 13 is not also limited, and a well-known material such as a resin material may be employed as necessary.

In the outer peripheral surface of the connection unit 13, a gas intake hole 13c communicating with the inside of the discharging unit 15 is opened. The gas intake hole 13c functions as an aperture through which the gas (air) flows into the discharging unit 15 brought under a negative pressure when the discharging unit 15 (in the state of FIG. 1(b)) pressed by the pressing operation by the surgeon or the like returns to the state (FIG. 1(a)) before the pressing operation, for example.

Note that the aperture through which the gas (air) flows into the discharging unit 15 brought under the negative pressure by the push may be provided in an end portion 15a of the discharging unit 15, for example. For example, the surgeon may perform the pressing operation while closing the aperture provided in the end portion 15a, thereby pressing the discharging unit 15 to discharge the gas in such a discharging unit into the chamber 11 through the protrusion 10. Then, the surgeon may open the closed aperture to return the pressed discharging unit 15 to the state before the pressing operation, thereby causing the gas (air) to flow into the discharging unit 15 brought under the negative pressure. With this configuration, the administration device 1 for discharging the medical powder onto the target site can be provided, which is operable with a simpler configuration.

Note that a structure that limits a gas inflow direction to one direction, such as a two-way valve, may be provided on the connection unit 13. Such a structure that limits the gas inflow direction to the one direction is configured, for example, to close between the gas intake hole and the path communicating with the inside of the discharging unit 15 when the gas is discharged into the chamber 11 from the pressed discharging unit 15 through the protrusion 10. As such a structure, various well-known structures such as diaphragm, ball, and disk type structures may be employed.

The finger grips 14 are a pair of members on which fingers are hooked when gripping the administration device 1. The finger grips 14 are formed in an annular shape, and are disposed at opposing positions on the outer peripheral surface of the chamber 11. As shown in FIG. 2(a), for example, the surgeon using the administration device 1 inserts the index finger Z2 and the middle finger Z3 into a pair of finger grips 14 formed in the annular shape, and grips the chamber 11 of the administration device 1 so as to pinch the chamber 11 between the facing positions. Then, when pressing the discharging unit 15, the surgeon presses the discharging unit 15 in the tip end direction with a thumb Z1 contacting the end portion 15a. Since the operating hand's index finger Z2 and middle finger Z3 are inserted into the pair of finger grips 14 formed in the annular shape, reliability when the administration device 1 is gripped can be enhanced. Moreover, since the discharging unit 15 can be pressed in the tip end direction with pressing force between the thumb Z1 and each of the index finger Z2 and the middle finger Z3, the pressing operation can be easily performed. The administration device 1 can be gripped with the index finger Z2 and the middle finger Z3 inserted into the pair of finger grips 14 formed in the annular shape, and therefore, when the pressed discharging unit 15 is returned to the state before the pressing operation, the push can be loosened by releasing the thumb Z1 contacting the end portion 15a. Note that the shape of the finger grip 14 is only required to be such a shape that the surgeon can pinch the chamber 11 from the opposing positions with the fingers hooked on the finger grip 14, and may be a flat plate shape or an arc shape.

In order to dispose the finger grips 14, for example, a pair of cut grooves for locking the finger grips 14 is formed at opposing positions in the outer periphery of the connection unit 13 engaged with the base end side of the chamber 11, and the finger grips 14 are formed with locking ridges extending in the base end direction. The locking ridges formed on the finger grips 14 are fitted in the locking grooves formed in the outer peripheral surface of the connection unit 13, and in this manner, the pair of finger grips 14 can be disposed at the opposing positions on the outer peripheral surface of the chamber 11. As a method for locking the finger grips 14, a well-known locking method may be employed as necessary. The finger grips 14 may be bonded and fixed to the opposing positions on the outer peripheral surface of the chamber 11. The material of the finger grips 14 is not limited, and a well-known material such as a resin material may be employed as necessary. Note that in a case where the material of the finger grip 14 is the same synthetic resin material as that of the connection unit 13, the finger grips 14 and the connection unit 13 may be integrally formed by injection molding, extrusion molding, or the like.

The discharging unit 15 is a member having a bellows-like column shape and formed extendable and contractable in the axial direction. The base-end-side end portion 15a of the discharging unit 15 is closed, and, when pressed, the tip end side engaged with the connection unit 13 is configured to discharge the compressed gas into the chamber 11 through the protrusion 10. Since the discharging unit 15 is formed in the bellows-like shape, the discharging unit 15 is extendable and contractable in the axial direction. For example, when the discharging unit 15 is pressed from the state shown in FIG. 1(a) to the state of FIG. 1(b) in response to the pressing force on the end portion 15a, the gas in the bellows-like portion is discharged into the chamber 11 through the protrusion 10. When the discharging unit 15 is extended from the state shown in FIG. 1(b) to the state of FIG. 1(a) by releasing the pressing force on the end portion 15a, the gas (air) flows into the bellows-like portion under the negative pressure through the gas intake hole 13c. The discharging unit 15 repeats a state change by the push to the tip end side by the pressing force and the extension (return) to the base end side by the release of the pressing force, thereby functioning as a pressing pump and serving as a gas supply source that supplies the gas pressed into the chamber 11. Hereinafter, the gas supplied from the discharging unit 15 will also be referred to as "pressurized gas".

In addition to the pressing pump such as a bellows-like pump and a rubber ball, a gas cylinder, an air pump, or the like may be used as the discharging unit. In a case where the gas cylinder is used as the discharging unit, a gas pressure regulation valve is preferably provided.

In a case where the pressing pump is used as the discharging unit 15, a well-known material such as polyolefin may be employed as necessary as the material thereof, for example. In a case where the discharging unit 15 is the bellows-like pump, the material thereof is preferably polyester or polyolefin, more preferably polyolefin, and much more preferably polyethylene. Note that the shape, size, and the like of the discharging unit 15 may be set as necessary according to the material and the like thereof.

The administration device 1 housing the medical powder in the chamber 11 is engaged with applicator 20, and functions as a medical implement. At least a tip end portion of the applicator 20 is inserted into a living body. The surgeon grips the administration device 1 with the fingers of the operating hand hooked on the pair of finger grips 14, and presses the end portion 15a of the discharging unit 15 formed in the bellows-like shape to the tip end side. The discharging unit 15 is pressed to the tip end side with the pressing force on the end portion 15a, and the gas in the bellows-like portion is discharged into the chamber 11 through the opening formed in the protrusion 10. The pressurized gas discharged into the chamber 11 flows into the applicator 20 engaged with the outlet unit 12 together with the medical powder while stirring the housed medical powder. The medical powder housed in the chamber 11 is discharged, together with the pressurized gas, onto the target site through the tubelike applicator 20.

The mechanism of action of the medical powder discharged onto the target site will be described taking the adhesive barrier described in Patent Literature 5 as an example. The adhesive barrier is a medical composition used for the purpose of preventing adhesion between a wound site and a surrounding tissue which may adhere to the wound site after the surgery. It is considered that the adhesion occurs when the tissue surface of the wound site and the surrounding tissue surface are joined or stuck to each other due to leakage/deposition of fibrin-containing exudate on the tissue surface of the wound site, for example. For example, in a laparotomy, when an intestinal tract in the abdominal cavity and an abdominal wall or intestinal tracts adhere to each other, mobility of the intestinal tract is harmed, which may cause intestinal obstruction (adhesive intestinal obstruction). For the purpose of preventing such adhesion, the adhesive barrier 90 which is the medical powder housed in the administration device 1 is discharged to a site between a wound site of an organ Z5 in the abdominal cavity and an abdominal wall Z4 which may adhere to the wound site, as shown in FIG. 2(b). The adhesive barrier 90 discharged from the applicator 20 is sprayed onto the surface of the wound site of the organ Z5 and the surface of the abdominal wall Z4 such as a peritoneal membrane.

The sprayed adhesive barrier 90 swells by absorbing moisture at the periphery of the wound site of the organ Z5. The swollen adhesive barrier 90 functions as a physical partition wall isolating the surface tissue of the wound site of the organ Z5 and the surface tissue of the abdominal wall Z4, which may adhere to the wound site, from each other and preventing the above-described adhesion between these tissues. Note that the swollen adhesive barrier 90 is subsequently brought into a solution state and gradually absorbed or decomposed, and can prevent the adhesion between the tissues.

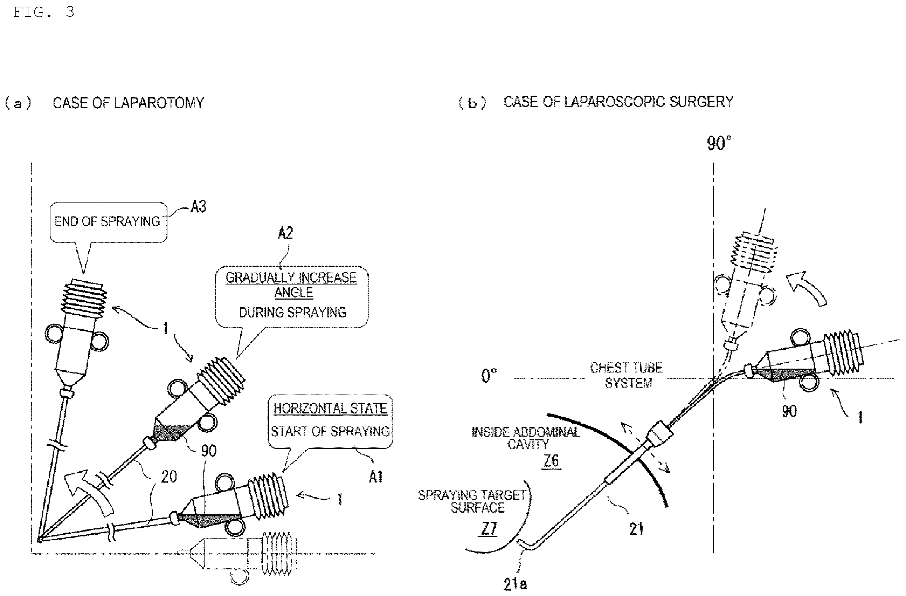

FIG. 3 shows views for describing the operation posture of the administration device 1 housing the adhesive barrier 90. FIG. 3(a) shows a schematic view for describing the operation posture of the administration device 1 in the laparotomy, and FIG. 3(b) shows a schematic view for describing the operation posture of the administration device 1 in a laparoscopic surgery. As shown in FIG. 3(a), in the laparotomy, the adhesive barrier 90 is sprayed onto the target site such as a peritoneal membrane through the applicator 20. The surgeon starts spraying the adhesive barrier 90 onto the target site in a state in which the administration device 1 housing the adhesive barrier 90 is in a horizontal state (balloon A1), for example. Here, the horizontal state indicates a state in which the axial direction along the center axis ZS1 of the administration device 1 is parallel with the horizontal direction. The surgeon sprays the adhesive barrier 90 while gradually lifting the administration device 1 about the tip end of the applicator 20 facing the target site (balloon A2). The posture of the administration device 1 is inclined such that the angle thereof relative to the horizontal direction gradually increases. As the adhesive barrier 90 housed in the chamber 11 decreases, the posture of the administration device 1 is inclined in a direction in which the angle of the administration device 1 relative to the horizontal direction increases. Then, the spraying ends (balloon A3).

In terms of a change in the posture of the administration device 1, the same also applies to spraying of the adhesive barrier 90 in the laparoscopic surgery. Note that the spraying of the adhesive barrier 90 in the laparoscopic surgery is performed through an applicator 21 invaded into the abdominal cavity Z6 of the living body using a chest tube system. The applicator 21 is a tubelike needle for the laparoscopic surgery, and a tip end portion 21a thereof is formed in a curved shape. For example, the surgeon operates the administration device 1 with which the applicator 21 is engaged while viewing an image of the inside of the abdominal cavity Z6 captured through a laparoscope, thereby causing the tip end portion 21a to face a spraying target surface Z7 of the peritoneal membrane or the like, which is the target site of the living body. Then, the pressing operation is performed while the posture of the administration device 1 is being inclined, so that the adhesive barrier 90 housed in the chamber 11 is sprayed onto the target site. The inclination of the administration device 1 relative to the horizontal direction from the start of the spraying to the end of the spraying also changes within an angle range of approximately 0° to 90° in the laparoscopic surgery.

### (Protrusion Structure)

Next, the structure of the protrusion 10 according to the present embodiment will be described with reference to FIGS. 4 and 5. FIG. 4 shows perspective views for describing the structure of the protrusion 10, and FIG. 5 shows perspective views for describing other structures of the protrusion 10. FIGS. 4(a), 4(b), 5(a), and 5(b) show, as examples, different forms of the structure of the protrusion 10.

As shown in FIG. 4(a), the protrusion 10 has a tubular structure extending from the base end side to the tip end side in the chamber 11 and protruding into the space where the medical powder can be housed. For example, the protrusion 10 is formed axisymmetrically about the center axis ZS1 shown in FIGS. 1(a) and 1(b). A tip-end-side protruding end 10b of the protrusion 10 is closed, and in the side surface thereof, an opening 10a is opened in the circumferential direction about the axial direction. The opening 10a is formed so as to communicate with a space 10c formed in the protrusion 10. The space 10c formed in the protrusion 10 has an inlet port on the connection unit 13 side, and is inserted so as to communicate with a connection space 13a in the connection unit 13. Further, the connection space 13a in the connection unit 13 is inserted so as to communicate with the inside of the bellows-like portion of the discharging unit 15. The protrusion 10 has such a structure so that the pressurized gas generated as a result of the push of the discharging unit 15 can be discharged into the space in the chamber 11 housing the medical powder through the opening 10a.

The material of the protrusion 10 is not limited, and any of well-known materials such as a glass material and a resin material may be employed. However, the same material as that of the connection unit 13, such as resin, is preferable. In a case where the material of the protrusion 10 is a synthetic resin material, the protrusion 10 can be molded in an arbitrary shape by injection molding, extrusion molding, or the like.

The opening 10a opened in the side surface of the protrusion 10 may include one opening or two or more openings. In the case of one opening, the gas pressed by the push of the discharging unit 15 can be discharged, with relatively-high pressing force, to a bottom-side region where the medical powder housed in the chamber accumulates. In a case where the openings 10a are provided at the plurality of locations in the axial direction, the pressed gas can be discharged to a plurality of bottom-side regions where the medical powder housed in the chamber is collected. The medical powder collected on the bottom side in the chamber 11 swirls in the air in the chamber 11 by the discharged pressurized gas. The swirling medical powder is diffused in the chamber 11, and is discharged to the outlet unit 12 together with the pressurized gas. Further, in a case where the openings 10a are provided at the plurality of locations in the circumferential direction about the axial direction as shown in FIGS. 4(b), 5(a), and 5(b), a uniform flow of the pressurized gas causing the medical powder, which is stirred and swirls in the air in the chamber 11, to flow toward the outlet unit 12 along the inner peripheral surface of the chamber 11 can be formed.

The hole area (area per hole) of the opening 10a may be adjusted according to the particle size of the medical powder, but for example, is preferably 1 mm² or less and more preferably 0.5 mm² or less. The hole area (area per hole) of the opening 10a is preferably 0.01 mm² or more and more preferably 0.1 mm² or more. The hole shape of the opening 10a is not particularly limited, and for example, may be a circular shape, a quadrangular shape, a semicircular shape, or the like.

In the space in the chamber 11, the base end side of the protrusion 10 will be referred to as a proximal end, and the outlet unit 12 side will be referred to as a distal end. The space in the chamber 11 has a distance extending from the proximal-end-side inner surface (i.e., surface facing the outlet unit 12) to the distal-end-side inner surface (inner surface on the outlet unit 12 side). In such a space in the chamber 11, the opening 10a of the protrusion 10 is preferably opened at a position with a distance of 1/3 or more in the direction from the surface facing the outlet unit 12 toward the outlet unit 12. With this configuration, the opening 10a of the protrusion 10 can be opened at a position relatively close to the outlet unit 12 in the chamber 11. Thus, as compared to the case of an opening on the base end side, the medical powder housed in the chamber 11 is much more easily discharged from the outlet unit 12. Even when the posture of the administration device 1 is inclined, the medical powder housed in the chamber 11 can be stably sprayed.

The protrusion 10 may be formed integrally with or separately from the chamber 11 or the connection unit 13. The protrusion 10 is formed separately so that the replaceable protrusion 10 can be provided as necessary according to the type, material, and the like of the medical powder housed in the chamber 11, for example.

FIG. 4(b) shows one example of the form in which the protrusion 10 is formed separately. Note that for this form, the engagement by the interference fit will be described, but a well-known engagement method such as screwing may be employed as necessary. A well-known material may be employed as the material of the protrusion 10 formed separately, but is preferably a resin material for which injection molding, extrusion molding, or the like can be employed. The quantity, size, shape, and the like of the opening 10a formed in the protrusion 10 may be set as necessary according to the type and material of the medical powder housed in the chamber 11.

The protrusion 10 formed separately has an engagement portion 10d formed engageable with the connection unit 13, and is formed in an axisymmetric tubular shape with the closed tip-end-side protruding end 10b. In the side surface of the protrusion 10, a plurality of openings 10a is opened in the circumferential direction about the axial direction, and each opening 10a communicates with the space 10c formed in the protrusion 10. In the connection unit 13, an engagement hole 13b formed engageable with the engagement portion 10d of the protrusion 10 is opened, and the engagement hole 13b is inserted so as to communicate with the connection space 13a of the connection unit 13. The engagement portion 10d of the protrusion 10 is fitted in the engagement hole 13b of the connection unit 13, and in this manner, the protrusion 10 formed separately and the connection unit 13 are engaged with each other by the interference fit. By the engagement, the space 10c formed in the protrusion 10 and the connection space 13a are inserted so as to communicate with each other. Even in this structure form, the pressurized gas generated as a result of the push of the discharging unit 15 can be discharged into the space in the chamber 11 housing the medical powder through the openings 10a and the connection space 13a inserted so as to communicate with the inside of the bellows-like portion of the discharging unit 15.

In the form of FIG. 4(b), the openings 10a opened in the side surface of the protrusion 10 are arranged at equal intervals of 90° in the circumferential direction. As indicated by dashed arrows, the protrusion 10 can discharge the pressurized gas into the space in the chamber 11 housing the medical powder at every 90° in the circumferential direction about the protrusion 10. With the pressurized gas discharged at every 90° about the protrusion 10, it can be expected that the uniformity of the flow generated toward the outlet unit 12 in the chamber 11 is enhanced and the stability of the medical powder discharged together with the pressurized gas is enhanced.

Note that the shape of the protrusion 10 protruding into the chamber 11 may be a circular columnar shape shown as an example in FIG. 4(a) and the like, or may be other shapes. For example, the protrusion 10 is only required to have an axisymmetric shape closed on the tip end side and have the space 10c inserted so as to communicate with the inside of the bellows-like portion of the discharging unit 15 and communicating with the openings 10a opened in the side surface of the protrusion 10. For example, the protrusion 10 may be formed in a rectangular columnar shape 10h shown as an example in FIG. 5(a), a pyramidal shape (e.g., conical shape or pyramid shape), or a truncated pyramidal shape (e.g., truncated conical shape 10i shown as an example in FIG. 5(b) or truncated pyramid shape). The shape may be set as necessary according to the material and size of the protrusion 10, the type and material of the medical powder housed in the chamber 11, a spraying amount necessary for a medical treatment, and the like. The protruding end of the protrusion 10 may be opened or closed. Since the flow rate of the pressurized gas discharged to the side surface of discharging unit is allowed to be higher, the protruding end of the protrusion 10 is preferably closed.

### (Spraying Test)

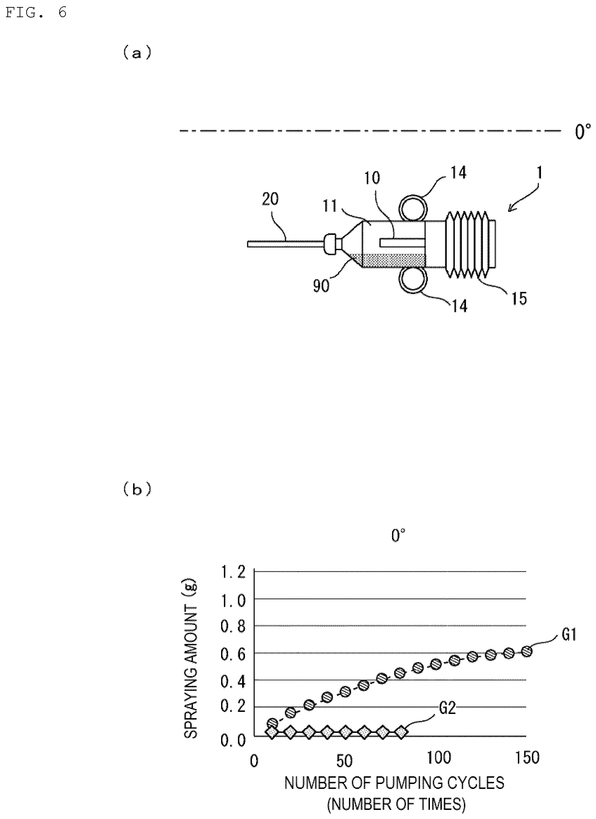

Next, steps and results of a test for the spraying of the medical powder with the administration device 1 will be described with reference to FIGS. 6 to 8. The spraying test was performed in a medical implement form in which the medical powder is housed in the chamber 11 of the administration device 1 and the applicator 20 (a tube inner diameter of 3.2 mm) used for the laparotomy or the like is attached to the outlet unit 12. As the medical powder related to the spraying test, the adhesive barrier 90 (particle size: 95% or more of the particles (in mass equivalent) have less than 0.5 mm) prepared according to Example 20 of Patent Literature 5 and containing the crosslinked chondroitin sulfate as a main component was employed, and the amount of the adhesive barrier 90 housed in the chamber 11 (an interior content of 24 mL) was 1 g. In the administration device 1, the quantity of the openings 10a opened in the circumferential direction in the side surface of the protrusion 10 was four at equal intervals of 90°, and the opening was in a circular shape with a hole area (per hole) of 0.4 mm². A spraying operation was performed in a state in which the administration device 1 is inclined at a certain angle without changing the posture of the administration device 1. The inclination angle of the administration device 1 was set to three angles which are an angle of 0 at which the administration device 1 is parallel with the horizontal direction and angles of 25 degrees and 40 degrees relative to the horizontal direction. In the spraying test, in a state of the inclination angle being held, the pressurized gas was discharged into the chamber 11 by extending and contracting the bellows-like portion of the discharging unit 15, and the weight of the medical powder discharged from the applicator 20 together with the pressurized gas was measured. The weight of the medical powder was measured every ten motions with one reciprocating motion of the push and the return as the unit of a pump operation.

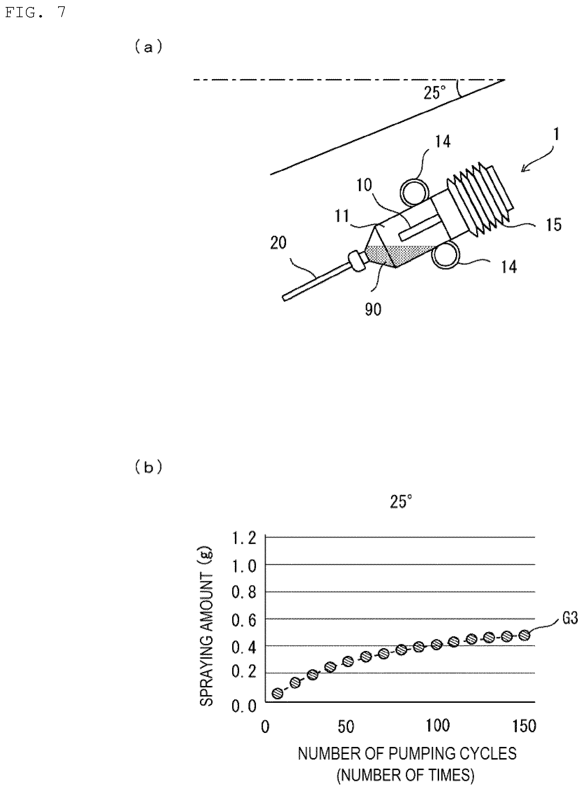

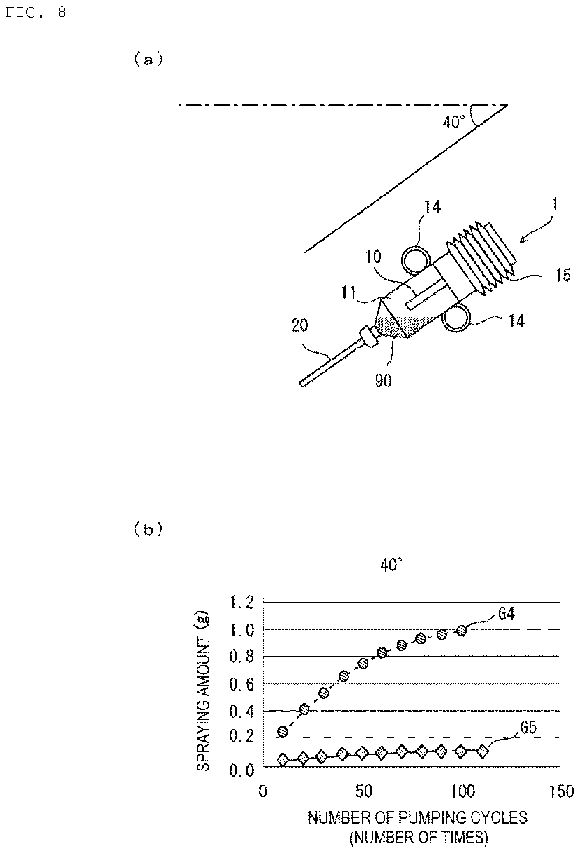

FIG. 6 shows views for describing the spraying test when the inclination angle of the administration device 1 is 0 degrees. Similarly, FIG. 7 shows views for describing the spraying test when the inclination angle of the administration device 1 is 25 degrees, and FIG. 8 shows views for describing the spraying test when the inclination angle of the administration device 1 is 40 degrees. Each (a) of FIGS. 6 to 8 shows a view for describing the inclination of the posture of the administration device 1 in the spraying test. A dash-dotted line in each (a) of FIGS. 6 to 8 indicates the horizontal direction. Each (b) of FIGS. 6 to 8 shows a graph of the results of the spraying test. In the graph showing the results of the spraying test in FIG. 6(b), the vertical axis indicates a spraying amount (g), and the horizontal axis indicates the number of cycles of the pump operation. The same also applies to FIGS. 7(b) and 8(b). Note that in order to evaluate the spraying amount of the medical powder in the spraying test when the inclination angle of the administration device 1 is 0 degrees and 40 degrees, a similar operation was performed on an administration tool (administration device) formed with no protrusion 10 to measure the spraying amount.

In FIG. 6(b), a graph G1 is a graph showing a transition in the spraying amount in association with the number of pumping cycles with the administration device 1, and a graph G2 is a graph showing a transition in the spraying amount in association with the number of pumping cycles with the administration tool as the comparative form. As shown in the graph G2, in a case where the administration tool formed with no protrusion 10 is operated and pressed in the horizontal state, the spraying amount of the medical powder discharged from the applicator 20 transitions around approximately 0 (g) regardless of the number of the pumping operation cycles. On the other hand, as shown in the graph G1, in the administration device 1 including the protrusion 10, a constant amount of medical powder housed in the chamber 11 can be discharged along with the pump operation of the discharging unit 15, and therefore, a stable spraying amount can be ensured even in the horizontal state.

A graph G3 of FIG. 7(b) is a graph showing a transition in the spraying amount in association with the number of pumping cycles with the administration device 1. Even in a case where the inclination angle of the administration device 1 is 25 degrees, a constant amount of medical powder housed in the chamber 11 can be discharged along with the pump operation of the discharging unit 15, and therefore, a stable spraying amount can be ensured. Note that the transition in the spraying amount of the medical powder discharged from the applicator 20 shows such a tendency that the gradient becomes gentler at around approximately 60 to 70 in terms of the number of pumping cycles. It is estimated that this is because the medical powder in the chamber 11 inclined at 25 degrees decreases as the number of pumping cycles increases and the amount of medical powder stirred by the pressurized gas discharged from the openings 10a of the protrusion 10 decreases.

In FIG. 8(b), a graph G4 is a graph showing a transition in the spraying amount in association with the number of pumping cycles with the administration device 1, and a graph G5 is a graph showing a transition in the spraying amount in association with the number of pumping cycles with the administration tool as the comparative form. In a case where the inclination angle is 40 degrees, a given amount of medical powder clusters in the vicinity of the outlet unit 12, and for this reason, a constant amount of medical powder can be discharged even in the comparative form formed with no protrusion 10 and the spraying amount is measured. However, as shown in the graph G5, even after the pumping has been repeated approximately 100 times, the spraying amount is extremely small. On the other hand, in the administration device 1 including the protrusion 10, a constant amount of medical powder housed in the chamber 11 can be discharged along with the pump operation of the discharging unit 15 as shown in the graph G4, and therefore, a stable spraying amount is ensured. Moreover, the gradient of the transition in the spraying amount of the medical powder tends to be gentler at around approximately 60 to 70 in terms of the number of pumping cycles, but all the medical powder housed in the chamber 11 is sprayed when the number of pumping cycles reaches approximately 100.

The present invention has been described in association with specific examples and various embodiments, but those skilled in the art easily understand that many modifications and applications of the embodiments described in the present specification can be made without departing from the spirit and scope of the present invention.

The present application claims priority to Japanese Patent Application No. 2023-036835 filed to the Japan Patent Office on March 9, 2023, the contents of which are incorporated by reference herein in its entirety.

### Reference Signs List

1: Administration device
10: Protrusion
10a: Opening
10b: Protruding End
10c: Space
10d: Engagement Portion
10h: Rectangular Columnar Shape
10i: Truncated Conical Shape
11: Chamber
12: Outlet Unit
13: Connection Unit
13a: Connection Space
13b: Engagement Hole
13c: Gas Intake Hole
14: Finger Grip
15: Discharging Unit
15a: End Portion
20, 21: Applicator
90: Adhesive Barrier

## Claims

1. An administration device for medical powder, comprising:
a chamber having an outlet port through which the medical powder is discharged and having a space where the medical powder is housable;
a protrusion protruding into the space from a surface facing the outlet port to the outlet port in the chamber and having an opening in a side surface of the protrusion; and
a discharging unit that discharges gas from the opening into the space, through an inlet port of the protrusion communicating with the opening from a side opposite to a tip end of the protrusion.

2. The administration device according to claim 1, wherein
the protrusion is axisymmetric, and the opening is opened in a circumferential direction about an axial direction in the side surface in the vicinity of the tip end.

3. The administration device according to claim 1 or 2, wherein
a shape of the protrusion is a pyramid shape, a conical shape, a rectangular columnar shape, or a circular columnar shape.

4. The administration device according to any one of claims 1 to 3, wherein
a protruding end of the protrusion is closed.

5. The administration device according to any one of claims 1 to 4, wherein
the opening is opened at a position with a distance of 1/3 or more of a distance between the surface facing the outlet port and an inner surface on the outlet port side inside the chamber in a direction from the surface facing the outlet port toward the outlet port.

6. The administration device according to any one of claims 1 to 5, further comprising:
a finger grip on which a finger is hooked when a user grips the administration device.

7. The administration device according to any one of claims 1 to 6, wherein
the discharging unit is a pressing pump.

8. The administration device according to any one of claims 1 to 7, wherein
the opening includes openings opened at an equal interval at a plurality of locations in the circumferential direction about the axial direction in the side surface in the vicinity of the tip end.

9. The administration device according to any one of claims 1 to 8, wherein
a hole area of the opening is 1 mm² or less per hole.

10. A medical implement comprising:
the administration device according to any one of claims 1 to 9, which has the chamber housing the medical powder.

11. The medical implement according to claim 10, wherein
the medical powder is an adhesive barrier.

12. The medical implement according to claim 10 or 11, further comprising:
a tubular applicator.

13. A surgical method using the medical implement according to claim 12, comprising:
inserting at least a tip end portion of the applicator connected to the outlet port into a living body; and
discharging gas from the tip end portion to spray the medical powder onto a target site of the living body together with the gas.

14. The surgical method according to claim 13, wherein
a surgery is a laparotomy or a laparoscopic surgery.

15. The surgical method according to claim 13 or 14, wherein
the target site is a peritoneal membrane.
